# EUROPEAN PATENT APPLICATION

(11) **EP 3 885 431 A1**
(43) Date of publication of application: **29.09.2021**
(21) Application number: 20382238.2
(22) Date of filing: 26.03.2020
(51) Int. Cl.: C12N 1/20, C12P 19/04, A61K 31/715, A61P 31/00

(54) **ANTIVIRAL COMPOSITION**

(71) Applicant: Universidad Autónoma de Madrid, 28049 Madrid (ES)
(72) Inventor: ABRUSCI BERNAL, Concepción, 28049 Madrid (ES); BELLO-MORALES ARROYO, Raquel, 28049 Madrid (ES); SÁNCHEZ LEÓN, Enrique, 28049 Madrid (ES); LÓPEZ GUERRERO, José Antonio, 28049 Madrid (ES)
(74) Representative: Hoffmann Eitle

(57) **Abstract**

**ANTIVIRAL COMPOSITION**

Antiviral composition. The present invention refers to the use of the bacterial strain *Bacillus licheniformis* characterized by the 16S rDNA of SEQ ID NO: 1 for obtaining a polymer. Moreover, the present invention refers to an antiviral pharmaceutical composition comprising the polymer obtained from the bacterial strain. The antiviral pharmaceutical composition of the invention is particularly suitable for the treatment of viral infections caused by enveloped virus.

## Description

### FIELD OF THE INVENTION

The present invention refers to the medical field. Particularly, the present invention refers to the use of the bacterial strain *Bacillus licheniformis* characterized by the 16S rDNA of SEQ ID NO: 1 for obtaining a polymer with antiviral activity. Moreover, the present invention refers to an antiviral pharmaceutical composition comprising the polymer obtained from the bacterial strain. The antiviral pharmaceutical composition of the invention is particularly suitable for the treatment of viral infections caused by enveloped virus.

### STATE OF THE ART

Enveloped viruses are viruses that have an outer wrapping or envelope. This envelope comes from the infected cell, or host, in a process called "budding off." During the budding process, newly formed virus particles become "enveloped" or wrapped in an outer coat that is made from a small piece of the cell's plasma membrane or also from internal membranes such as the trans-Golgi network. The envelope may play a role in helping a virus survive and infect other cells.

The lipid bilayer envelope of these viruses is relatively sensitive to desiccation, heat, and detergents; therefore, these viruses are easier to sterilize than non-enveloped viruses, have limited survival outside host environments, and typically must transfer directly from host to host.

Nevertheless, enveloped viruses possess great adaptability and can change in a short time in order to evade the immune system. Enveloped viruses can cause persistent infections. The group of enveloped viruses comprises a well-defined number of pathogenic human viruses such as *[*Dimitrov DS et al., 2004. Virus entry: molecular mechanisms and biomedical applications. Nat Rev Microbiol. 2004 Feb;2(2):109-22*. PMID: 15043007 DOI: 10.1038*/*nrmicro817*]:

| **Family** | **Representative virus** |
|---|---|
| Arenaviridae | Lassa fever virus (LFV) |
| Bornaviridae | Borna disease virus (BDV) |
| Bunyaviridae | Hantaan virus (HTNV) |
| Coronaviridae | SARS-CoV |
| Deltavirus | Human hepatitis delta virus Endocytosis Hepatitis (HDV) |
| Filoviridae | Ebola virus |
| Flaviviridae | Tick-borne encephalitis virus (TBE) |
| Hepadnaviridae | Hepatitis B virus (HBV) |
| Herpesviridae | Herpes simplex virus 1 (HSV-1) |
| Orthomyxoviridae | Influenza A virus (IV) |
| Paramyxoviridae | Human respiratory syncytial virus (hRSV) |
| Poxviridae | Smallpox |
| Retroviridae | HIV type 1 |
| Rhabdoviridae | Rabies virus |
| Togaviridae | Semliki Forest virus (SFV) |

Since, as explained above, enveloped viruses can cause persistent infections, there is an unmet medical need of finding antiviral compositions able to effectively treat viral infections caused by this specific virus type.

The present invention is focused on solving this problem a new antiviral pharmaceutical composition is herein provided.

### DESCRIPTION OF THE INVENTION

### Brief description of the invention

The present invention refers to the use of the bacterial strain *Bacillus licheniformis* characterized by the 16S rDNA of SEQ ID NO: 1 for obtaining a polymer (an exopolymer; EPSp) with antiviral activity. Moreover, the present invention refers to an antiviral pharmaceutical composition comprising the polymer obtained from the bacterial strain. The antiviral pharmaceutical composition of the invention is particularly suitable for the treatment of viral infections caused by enveloped virus.

### Method for obtaining a polymer

The first embodiment of the present invention refers to an *in vitro* method for obtaining a polymer with antiviral activity which comprises culturing the bacterial strain *Bacillus licheniformis* of SEQ ID NO: 1 under conditions conducive to produce the polymer. In a preferred embodiment the method comprises:
a. Incubating the bacterial strain *Bacillus licheniformis* characterized by the 16S rDNA of SEQ ID NO: 1 in suitable growth media;
b. Centrifugating the cultures obtained in step a);
c. Precipitating the supernatant obtained in step b);
d. Collecting the precipitate obtained from step c);
e. Dialyzing; and
f. Freeze drying.

In a preferred embodiment of the invention the incubating step a) comprises:
a. Transferring the strain into flasks filled with minimal growth medium comprising: 6.8 g/L Na₂HPO4, 3 g/L KH₂PO4, 1 gr/L NH₄Cl, 0.625 gr/L NaCl, 0.49 gr/L MgSO₄ · 7H₂O , 0.011 gr/L CaCl₂ and 4 gr/L Glucose,
b. Adjusting the pH to 7.0.
c. Incubating in a rotary at 45°C and 110 rpm for 24 hours.
d. Inoculating 10 ml of the minimal growth medium with the strain into flasks filled with 100 ml of minimal growth medium with Glucose supplementation.
e. Incubating the flasks in a rotary at 45°C and 110 rpm for 40 hours.

In a preferred embodiment of the invention the centrifugating step b) is carried out at 12000-14000 x g for 30 minutes at 4°C.

In a preferred embodiment of the invention the precipitating step c) is carried out with cold ethanol.

In a preferred embodiment of the invention the collecting step d) is carried out by centrifugation at 12000-14000 x g for 30 minutes at 4°C.

In a preferred embodiment of the invention the dialyzing step d) is carried out at 4°C with Milli-Q water for 48h.

In a preferred embodiment of the invention the freeze-drying e) is carried out by lyophilization for 48h.

### Polymer

The second embodiment of the present invention refers to a polymer obtained by the above-mentioned method. In other words, the inventors of the present invention have obtained a polymer from the strain *Bacillus licheniformis* characterized by the 16S rDNA of SEQ ID NO: 1, and they have demonstrated that this polymer has a strong antiviral effect, particularly in enveloped virus.

The inventors of the present invention have made considerable efforts to characterize the physical composition of the polymer. Such as it is indicated in the examples, it is proposed that the majority products of the samples are the following:
- Chain of polyglutamic acid (polymer of the amino acid Glu), of no determined length (n), this being the compound with the largest molecular weight 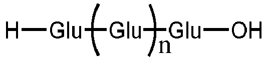
- Chain of polyglycerol phosphate with substitutions of αGalactose (Gal) in terminal positions and αGlucosamine (GlcNH₂). Due to the proportion Gal/GlcNH₂ (approximately 3:1) and taking into account the terminal position of the αGal, it is possible that some chains do not present αGlcNH₂.
- The mass spectrum of the polymer indicated that it had an approximate molecular weight of 4 to 6 KD (preferably 5kD).

This means that the polymer obtained from the strain *Bacillus licheniformis* characterized by the 16S rDNA of SEQ ID NO: 1 has a strong antiviral effect. So, the product (polymer) shows distinct differences in properties which implies a structural difference and causes for such antiviral properties.

In a preferred embodiment, the polymer substantially comprises a chain of polyglycerol phosphate with substitutions of Galactose and Glucosamine in a proportion 3:1 and a basic repeating unit of polyglutamic acid, wherein the molecular weight of the polymer is between 4 to 6 kD, preferably around 5 kD. In a preferred embodiment, the polymer is characterized in that it is produced by culturing the bacterial strain *Bacillus licheniformis* of SEQ ID NO: 1 under conditions conducive to produce the polymer.

### Pharmaceutical composition

The third embodiment of the present invention refers to a pharmaceutical composition comprising the above-mentioned polymer and optionally pharmaceutically acceptable excipients and/or carriers. In a preferred embodiment, the pharmaceutical composition is administered by any route known in the prior art for the administration of antiviral compositions. Oral, inhaled or hematogenous routes are the preferred routes of administration.

### Medial uses

The fourth embodiment of the present invention refers to the use of the above-cited pharmaceutical composition as a medicament, preferably in the treatment of viral infections caused by enveloped virus, and more preferably in the treatment of a viral infection caused by an enveloped virus selected from the group comprising: Herpes simplex virus (HSV-1 or HSV-2), Pseudorabies virus (PRV), Vesicular stomatitis virus (VSV) or Coronavirus. Alternatively, the present invention refers to a method for treating a viral infection, preferably a viral infection caused by an enveloped virus, which comprises the administration of a therapeutically effective amount of the pharmaceutical composition of the invention.

For the purpose of the present invention the following terms are defined:
- The term "comprising" is meant including, but not limited to, whatever follows the word "comprising". Thus, use of the term "comprising" indicates that the listed elements are required or mandatory, but that other elements are optional and may or may not be present.
- By "consisting of" is meant including, and limited to, whatever follows the phrase "consisting of'. Thus, the phrase "consisting of" indicates that the listed elements are required or mandatory, and that no other elements may be present.
- By "therapeutically effective dose or amount" is intended an amount that, when administered as described herein, brings about a positive therapeutic response in the patient. The exact amount required will vary from subject to subject, depending on (non-exhaustive list): the species, age, general condition of the subject, the severity of the condition being treated or the mode of administration. An appropriate "effective" amount in any individual case may be determined by one of ordinary skill in the art using routine experimentation, based upon the information provided herein.

### Description of the figures

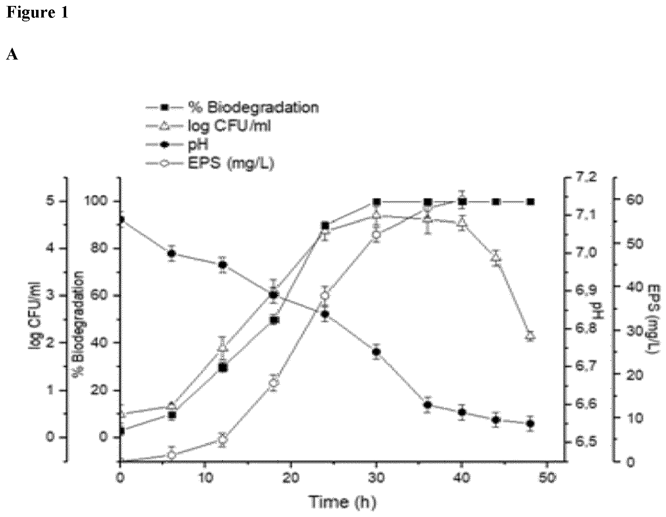

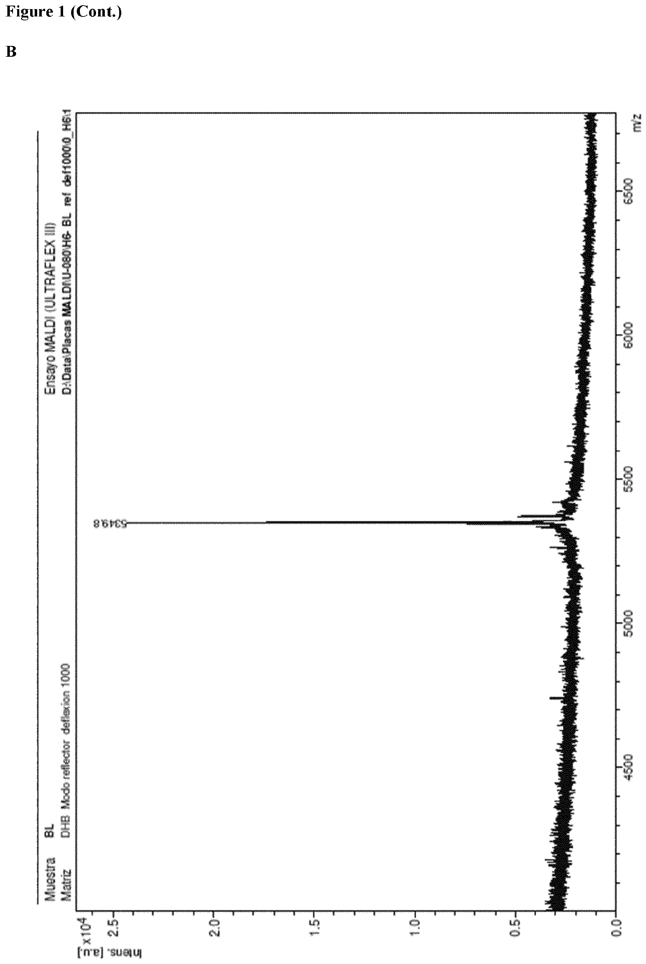

**Figure 1****. Characterization of the exopolymer EPSp extracted from *B. licheniformis* IDN-EC. A)** Time course of glucose biodegradation, colony-forming unit (CFU), pH value, and EPSp production at 45°C over time from 0 to 48 h. **B)** MALDI-TOF mass spectroscopy of EPSp.

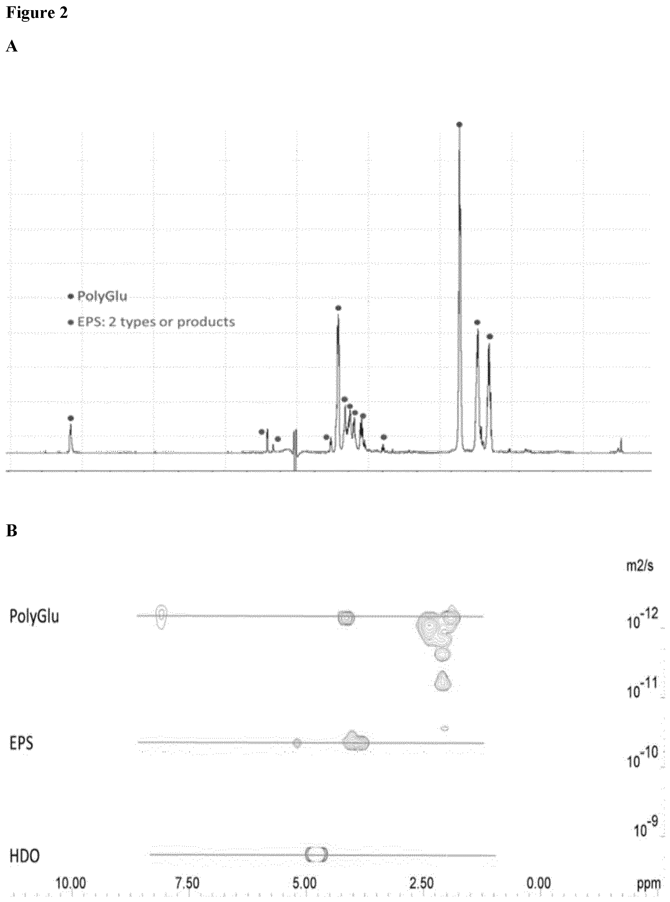

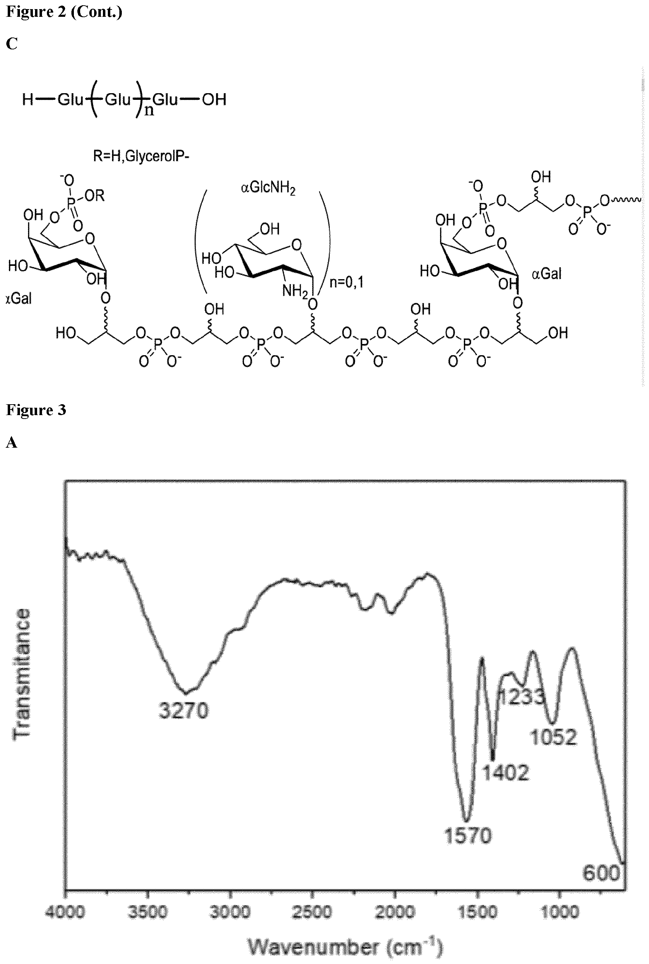

**Figure 2****. Nuclear magnetic resonance (NMR).** Characterization of the main parts of the exopolymer EPSp.

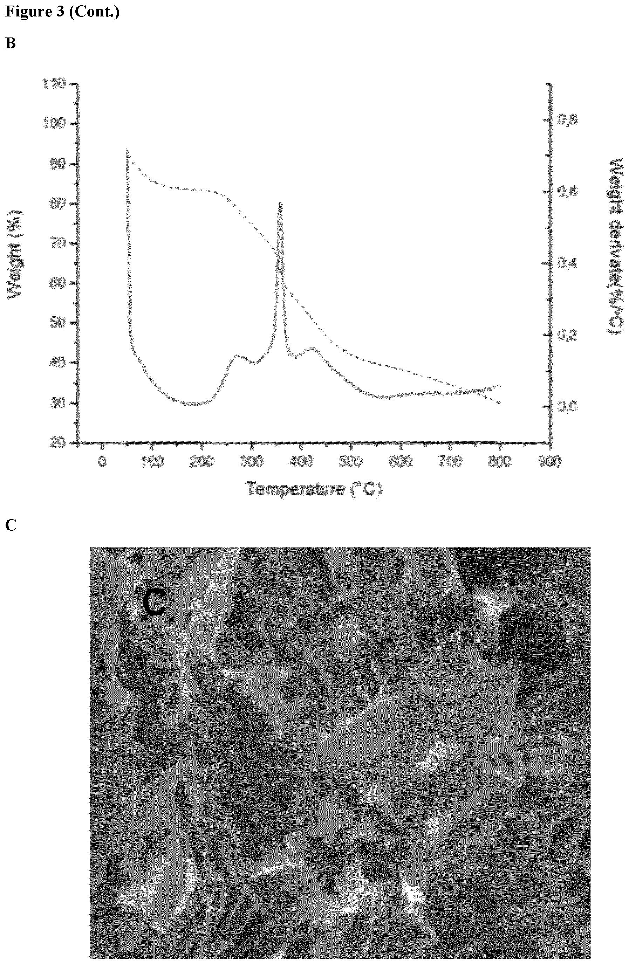

**Figure 3****. FTIR, thermal analysis and ultrastructural characterisation. A)** FTIR spectrum. **B)** Thermogravimetric analysis and DSC thermogram. C) Scanning electron micrographs.

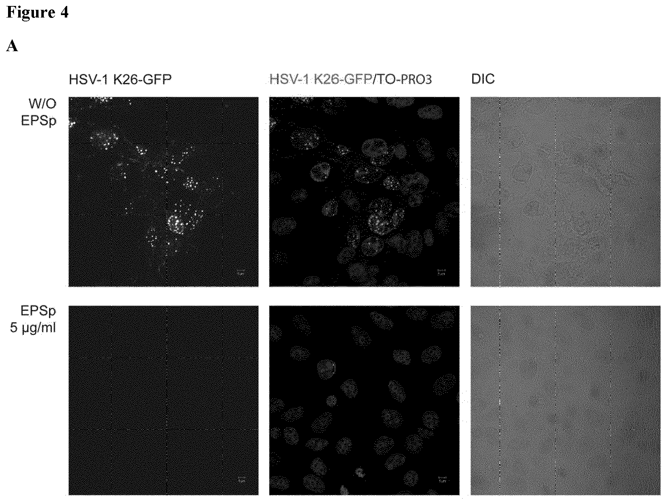

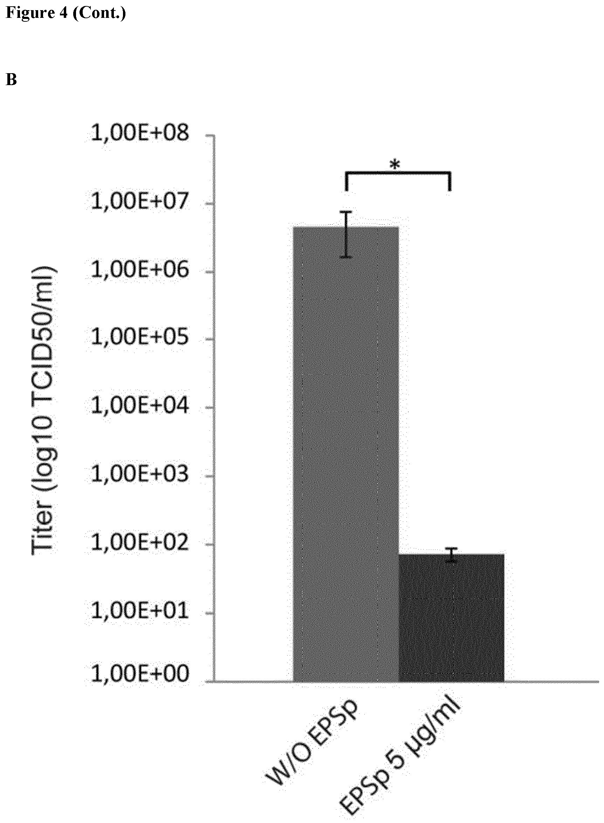

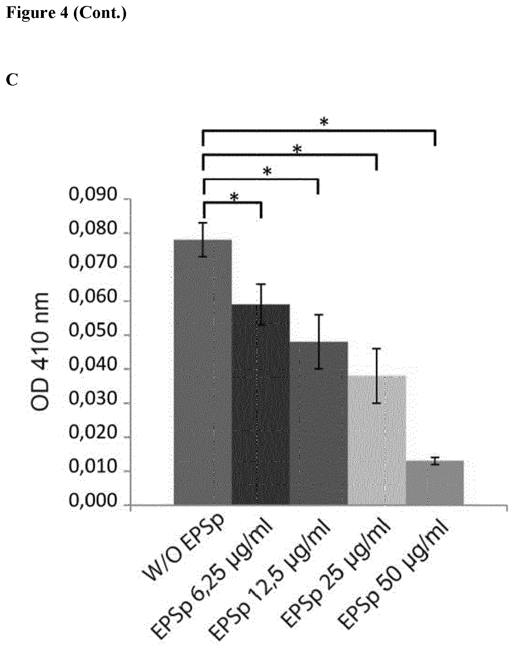

**Figure 4****. Exopolysaccharide EPSp of *B. licheniformis* IDN-EC drastically decreased HSV-1 infection of Vero cell line.** Cells were infected at an m.o.i. of 0.5 with HSV-1 K26-GFP pre-treated or mock pre-treated with EPSp (5 µg/ml) and cultured for 24 h at 37°C in a CO₂ incubator (**A, B**). **A.** Immunofluorescence images of cells show the GFP signal associated to HSV-1 K26. Nuclei were stained with TO-PRO 3. **B.** Progeny virus was titrated 24 h p.i. to determine the 50% tissue culture infective dose (TCID₅₀)/ml. Histogram shows the viral production cells infected with the virus pre-treated (blue bar) or mock pre-treated (green bar) with EPSp. **C.** Cells were infected at a m.o.i. of 10 with HSV-1 gL86 pre-treated or mock pre-treated with two-fold serial dilutions of EPSp 1 mg/ml, at final concentrations of 6.25, 12.5, 25 and 50 µg/ml. After 6 h p.i., the beta-galactosidase activity at 410 nm was analyzed in a microplate reader. (W/O EPS= without EPS pre-treatment; DIC: Differential Interference Contrast; * p<0.05).

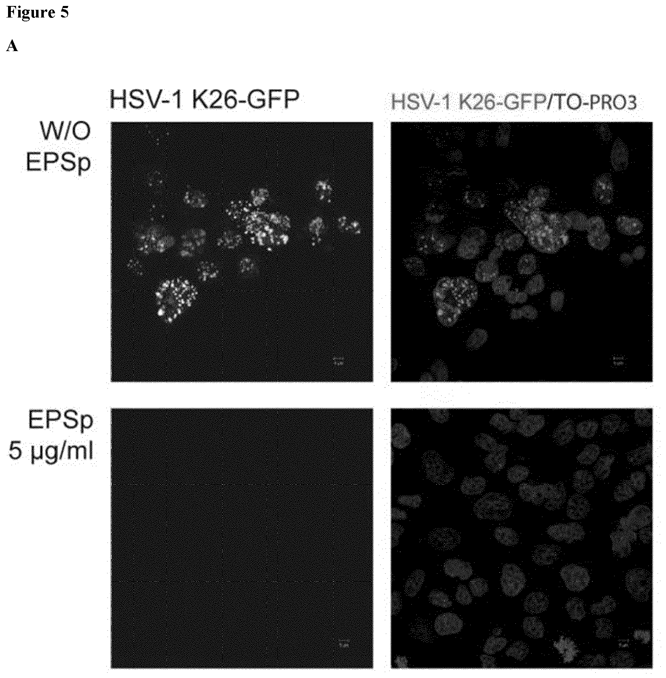

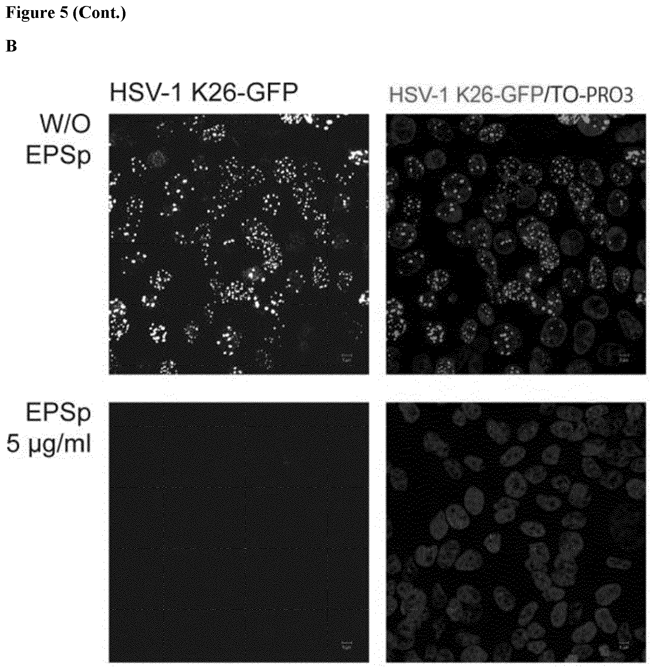

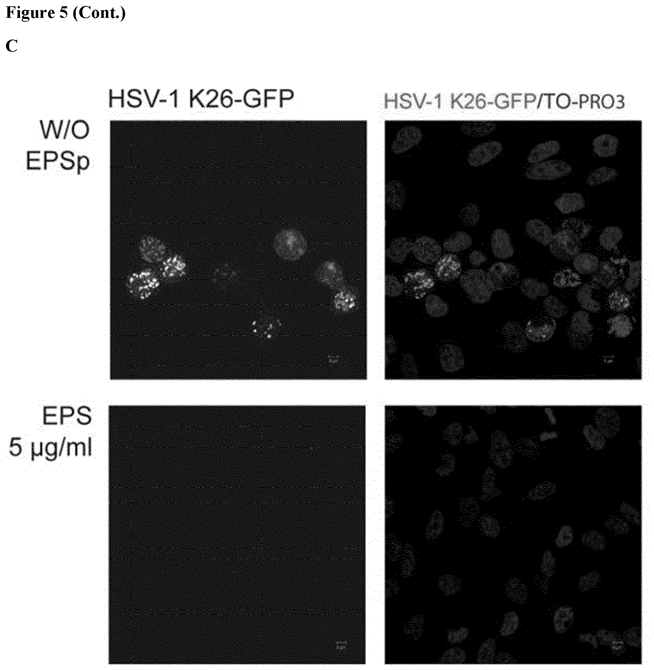

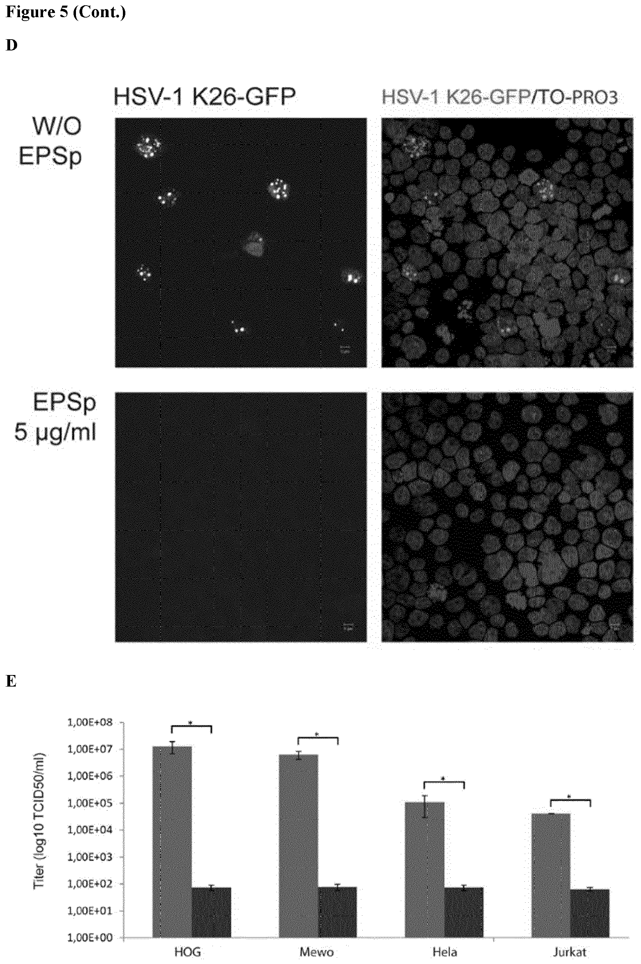

**Figure 5****. Effect of exopolysaccharide EPSp of *B. licheniformis* IDN-EC on HSV-1 infection of human cell lines.** HOG, Mewo, Hela and Jurkat cells were infected at an m.o.i. of 0.5 with HSV-1 K26-GFP pre-treated or mock pre-treated with EPSp (5 µg/ml) and cultured for 24 h at 37°C in a CO₂ incubator. Immunofluorescence images show the GFP signal associated to HSV-1 K26 in HOG (**A**), Mewo (**B**), Hela (**C**) and Jurkat (**D**) cells. Nuclei were stained with TO-PRO 3. **E.** Progeny virus was titrated 24 h p.i. to determine the 50% tissue culture infective dose (TCID₅₀)/ml. Histogram shows the viral production of HOG, Mewo, Hela and Jurkat cells infected with the virus pre-treated (blue bar) or mock pre-treated (green bar) with EPSp. (W/O EPSp= without EPSp pre-treatment; * p<0.05).

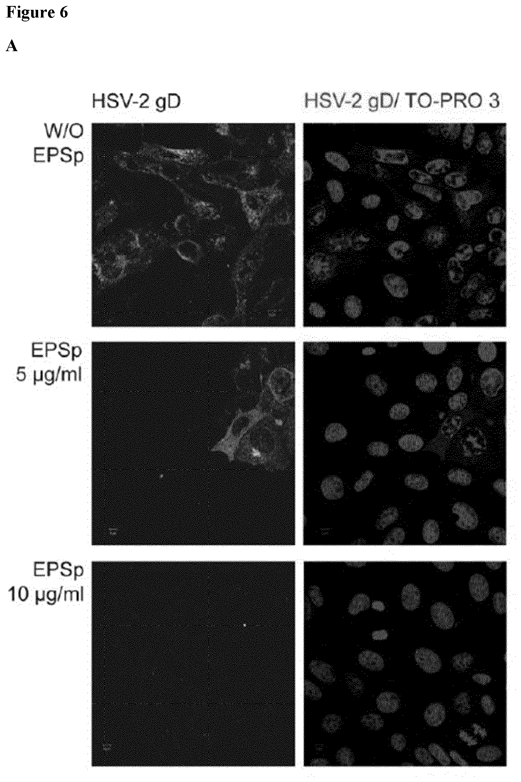

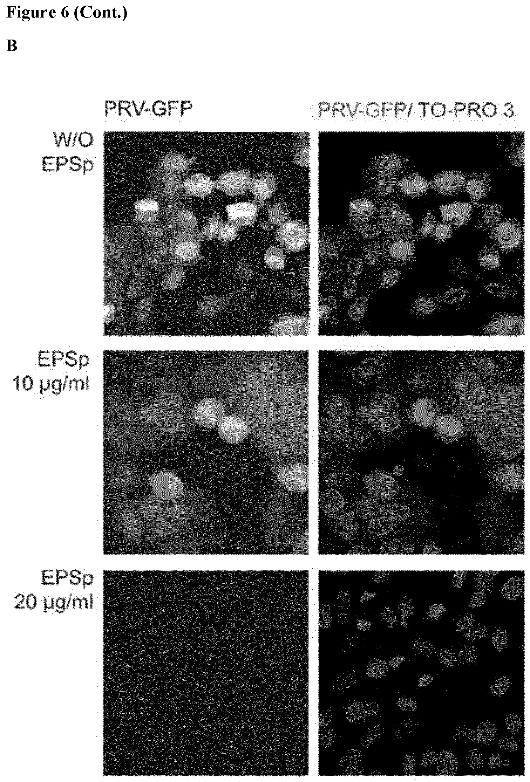

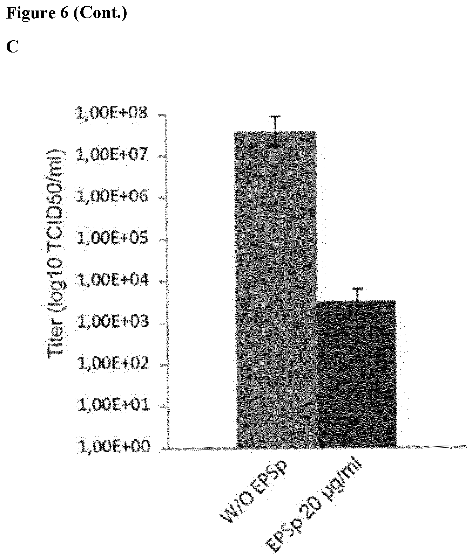

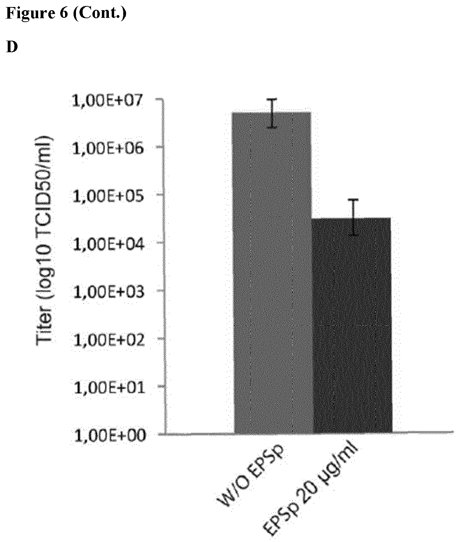

**Figure 6****. Exopolysaccharide EPSp of *B. licheniformis* IDN-EC drastically decreased HSV-2 and PRV-GFP infection of Vero cell line. A.** Vero cells were infected at an m.o.i. of 0.5 with HSV-2 pre-treated or mock pre-treated with EPSp (5 and 10 µg/ml) and cultured for 24 h at 37°C in a CO₂ incubator. Then cells were fixed and processed for confocal double-label indirect immunofluorescence analysis with the monoclonal anti-HSV gD LP2 antibody followed by an Alexa-555 donkey anti-mouse secondary antibody. Images correspond to the projection of confocal 0.6 µm planes. **B.** Vero cells were infected at an m.o.i. of 0.5 with PRV-GFP pre-treated or mock pre-treated with EPSp (10 and 20 µg/ml) and cultured for 24 h at 37°C in a CO₂ incubator. Then cells were fixed and processed for confocal double-label microscopy. Immunofluorescence images, which show the GFP signal associated to PRV, correspond to the projection of confocal 0.6 µm planes. Nuclei were stained with TO-PRO 3. **C-D.** Progeny virus was titrated 24 h p.i. to determine the 50% tissue culture infective dose (TCID₅₀)/ml. Histogram shows the viral production of Vero cells infected with HSV-2 (C) or PRV-GFP (D) pre-treated (blue bar) or mock pre-treated (green bar) with EPSp. (W/O EPSp= without EPSp pre-treatment).

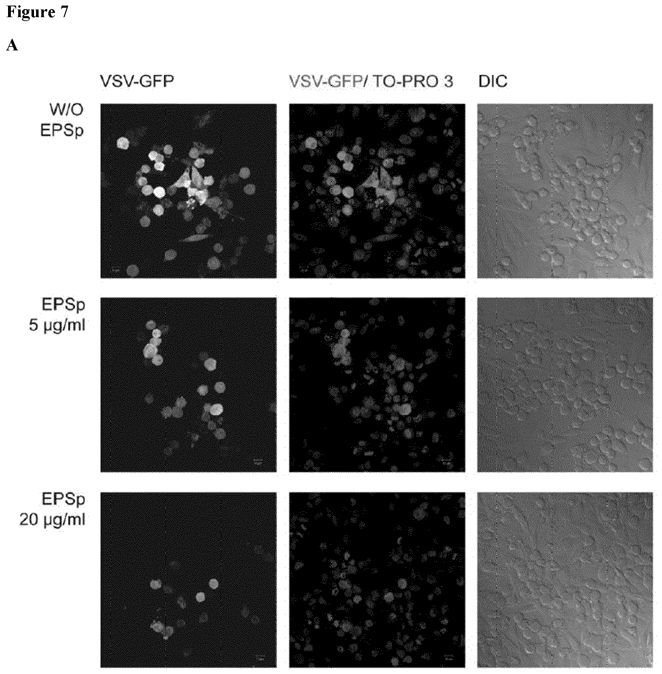

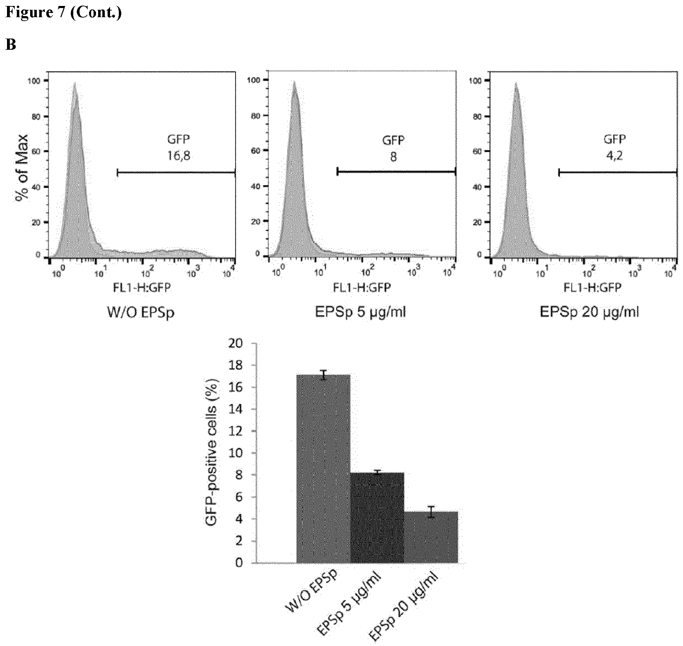

**Figure 7****. Effect of exopolysaccharide EPSp of B. licheniformis IDN-EC on VSV-GFP infection of Hela cell line.** Cells were infected at an m.o.i. of 0.5 with VSV-GFP pre-treated or mock pre-treated with EPSp (5 and 20 µg/ml) and cultured for 24 h at 37°C in a CO₂ incubator. Then cells were fixed and processed for confocal double-label indirect immunofluorescence analysis (**A**) or for flow cytometry (**B**). **A.** Images, which show the GFP signal associated to VSV, correspond to the projection of confocal 0.6 µm planes. Nuclei were stained with TO-PRO 3. **B.** Cells were processed for flow cytometry analyzing fluorescence of GFP. Plots show the percentage (%) of Max, which designates the number of positive cells relative to the maximum fraction. Data corresponding to three replicates are shown in the histogram. (W/O EPSp= without EPSp pre-treatment; DIC: Differential Interference Contrast; * p<0.05)

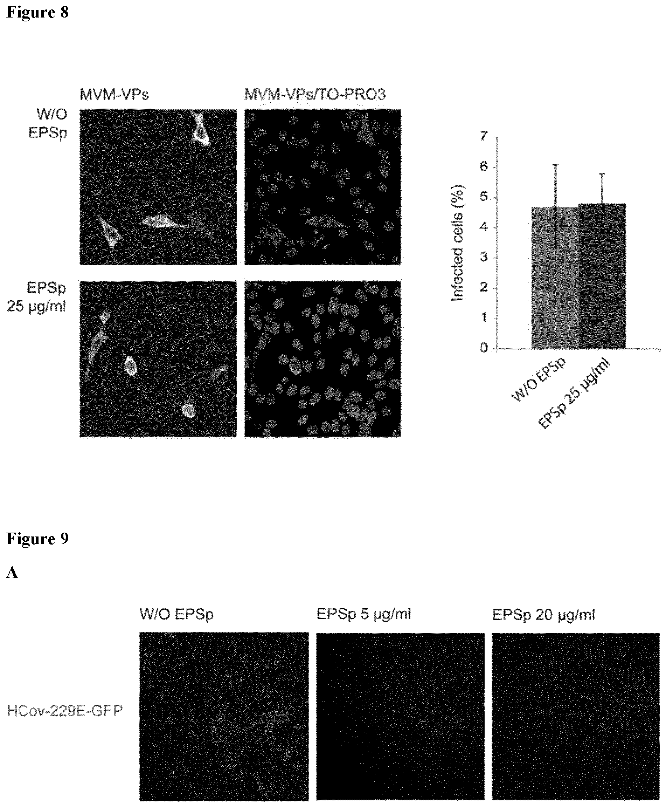

**Figure 8****. Effect of exopolysaccharide EPSp of *B. licheniformis* IDN-EC on MVM infection of Hela cell line.** Cells were infected at an m.o.i. of 0.5 with MVM pre-treated or mock pre-treated with EPSp (25 µg/ml) and cultured for 24 h at 37°C in a CO₂ incubator. Then cells were fixed and processed for confocal double-label indirect immunofluorescence analysis with the anti-VPs MVM polyclonal antibody followed by an Alexa-555 donkey anti-rabbit secondary antibody. Images correspond to the projection of confocal 0.6 µm planes. Nuclei were stained with TO-PRO 3. Histogram shows the quantification of immunofluorescence images. Bars correspond to the percentage of cells infected with virus pre-treated (blue) or mock pre-treated (green) with EPSp. (W/O EPSp= without EPSp pre-treatment).

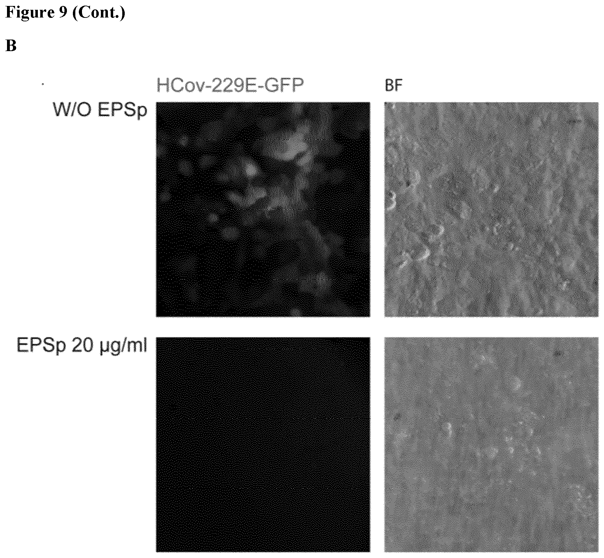

**Figure 9****. EPSp drastically decreased HCOV229-E-GFP infection of Huh7 cell line.** Cells were infected at an m.o.i. of 0.1 with HCov229-E-GPF pre-treated or mock pre-treated with EPSp (5 and 20 µ/ml) and cultured for 48 hours at 37°C in a CO₂ incubator. **A.** 20X objective. **B.** 40X objective (W/O EPSs: without EPSp).

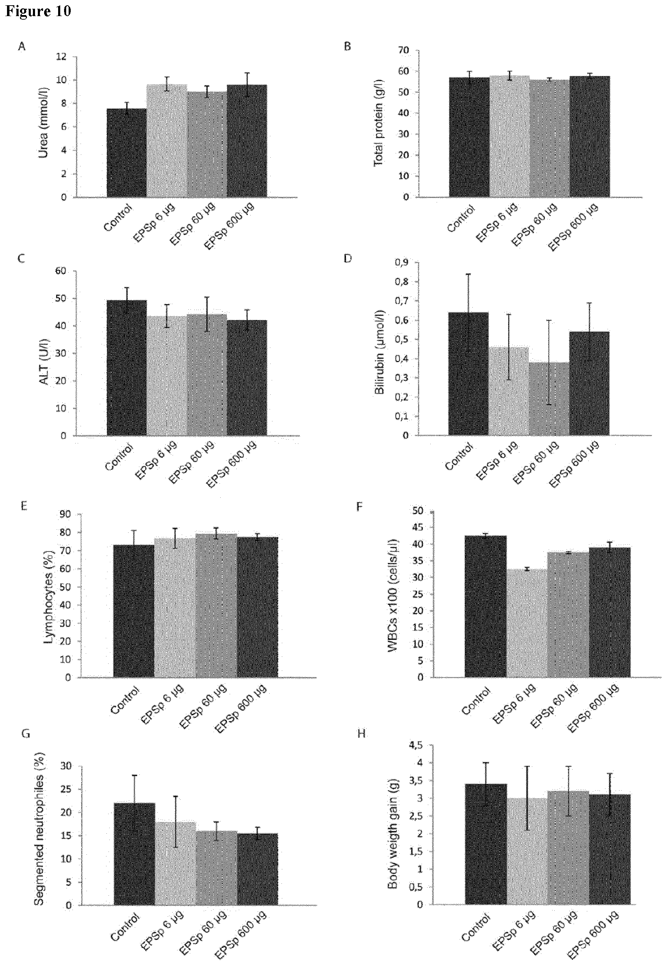

**Figure 10****. *In vivo* acute toxicity of EPSp in mice.** Twenty male Balb/c mice were randomly distributed in cages of 5 individuals and inoculated or mock-inoculated intraperitoneally with 100 µl of ten-fold concentrations of EPSp diluted in NaCl 0.9% w/v: 6, 60 and 600 µg of EPSp per animal. At day 14, mice were sacrificed and whole blood was obtained by cardiac puncture. For the biochemical analyses, urea (**A**), total protein (**B**), ALT (**C**) and bilirubin (**D**) levels were tested. For hematologic study, the percentages of lymphocytes (**E**), WBCs count (**F**) and segmented neutrophils (**G**) were analysed. The body weight gain was also monitored (H). Control: 100 µl of NaCl 0.9% w/v per animal. (ALT: alanine aminotransferase; WBC: white blood cells)

### Detailed description of the invention

The examples below have the purpose of illustrating the invention without limiting its scope.

### Example 1. Materials and methods.

### Example 1.1. Chemicals and standards.

Alexa555-conjugated secondary anti-mouse and anti-rabbit antibodies were from Molecular Probes (Eugene, OR, USA), and Mowiol was from Calbiochem (Merck Chemicals, Germany). The rest of reagents were purchased from Sigma Chemical Co. (St. Louis, MO, USA).

### Example 1.2. Bacterial strain

The indigenous bacterial strain, *Bacillus licheniformis* IDN-EC, Accession # (HM055601) (GeneBank) was used in this study. This strain had been isolated from films based on Poly(Butylene Adipate-co-Terephthalate) and its blend with Poly(Lactic Acid).

### Example 1.3. Production of exopolysaccharides

*Bacillus licheniformis* IDN-EC was inoculated from the stock culture in trypticase soya agar medium (TSA) and incubated at 45 °C for 24 h. After that, the strains were transferred into flasks of 100 ml filled with 20 ml of minimal growth medium (MGM) in g/L: Na₂ HPO₄, 6.8; KH₂PO₄, 3; NH₄Cl, 1; NaCl, 0.625; MgSO₄·7H₂O, 0.49; CaCl₂, 0.011, and glucose as a carbon source at a concentration of 4 g/L, pH adjusted to 7.0. The flasks were incubated in a rotary shaker incubator (Biogen) at 45°C and 110 rpm for 24 h. After the first incubation, 10 ml of this broth (2.5 × 10⁷ cells/ml concentration) was inoculated into flasks of 1000 ml filled with 100 ml of MGM with glucose supplementation. The flasks were incubated at 45°C and 110 rpm for 40 h, when the stationary phase was reached.

### Example 1.4. Biodegradation, cell growth, and pH

The biodegrading bacteria were evaluated by indirect impedance measurements. The aerobic biodegradation of glucose compound by *B. licheniformis* was performed at 45°C. The bioassays were carried out in bioreactors of 7 ml, filled with 1.5 mL of bacterial suspension prepared as described above. These containers were introduced into disposable cylindrical cells of 20 mL filled with 1.5 mL of 2 g/L KOH aqueous solution and provided by four stainless steel electrodes to measure impedance on a Bac-Trac 4300 apparatus (SY-LAB Geräte GmbH, Neupurkerdorf, Austria). The method has a typical error in the measurements of 1-2%. The experimental device and procedure have been previously described in the literature. The device monitors the relative change (each 20 min) in the initial impedance value of KOH solution, which is converted in concentration of carbon dioxide by a calibration curve of impedance variation versus concentration of CO₂. The percentage of biodegradation of glucose was calculated as a percentage of the ratio between the cumulative amount of CO₂ produced in the biodegradation at time, t, and the theoretical amount of carbon dioxide assuming that all the carbon of the glucose structures introduced in the bioreactor are transformed into CO₂. % Biodegradation = ([CO₂]Prod/[CO₂]Theor.) × 100.

The cell growth number was evaluated by different dilution plating incubated at 45°C for 48 h with TSA agar medium. A Thermo Orion pH Meter (model, 2 Star) was used to determine the pH values during a fermentation period of 48 h.

### Example 1.5. Isolation and purification of EPS

The cultures obtained from the strain *B. licheniformis* IDN-EC, were centrifuged at 13,154 × g for 30 min at 4°C (Duppont - RC5). The supernatant was precipitated with cold ethanol (three times the volume) and left overnight. The precipitate was collected by centrifugation at 13,154 x g for 30 min at 4°C and dissolved in Milli-Q water. Then the crude EPS was dialyzed at 4°C with Milli-Q water for 48 h. The dialyzed contents were then freeze dried by lyophilization for 48 h and the dry weight of the powdered EPS was determined.

The further purification of crude EPS (10 mL, 10 mg/mL) was subjected to a DEAE-52 anion exchange column (2.6×30 cm) and eluted with deionized water, 0.05 and 0.3 M NaCl at 1 mL/min flow rate. Each 10 mL of elution was collected and the phenol-sulfuric acid method (Dubois et al. 1956) was used to determine the polysaccharide content.

### Example 1.6. Mass spectrometry

MALDI-TOF mass spectra were recorded on an Ultraflex III TOF/TOF mass spectrometer (BrukerDaltonics) equipped with a Nd:YAG laser (355 nm). Mass spectra were recorded in positive reflector (range 1-10 KDa) and lineal (range 1-20 KDa) modes, using a matrix of 10mg/mL 2,5-dihydroxibenzoic acid (DHB) in methanol/water (90/10).

### Example 1.7. Monosaccharide Analysis

To determine the monosaccharide composition, the EPSp was hydrolysed with trifluoro acetic acid (TFA) 0,5M at 120°C for 2 h. The samples were treated before and after the process with N₂. The monosaccharide content of EPSₚ was analysed by HPLC using a 920LC Varian apparatus equipped with a PL-EDS 2100 Ice detector. A sugar SP0810 (Shodex) column as a stationary phase was used with an isocratic mobile phase of water as solvent and a flow rate of 0,5mL/min. The column temperature was maintained at 30°C. The samples injection volume was 50 µL. The mono sugars such as glucose, arabinose, rhamnose, xylose, mannose, galactose, fructose and sorbose were used as standards. Three independent assays were performed.

### Example 1.8. RNM Spectroscopy

For structural assignments of the isolated polymer, NMR spectra were recorded in D₂O by using a Bruker 600-800 MHz spectrometer equipped with a reverse cryo-probe with gradients along the z-axis. The NMR sequences used for structural elucidation were: (1H1H homonuclear) DQ-COSY (double quantum COSY spectrum, hereafter referred as COSY), TOCSY, NOESY, ROESY and DOSY (1H13C heteronuclear) HSQC, and HSQC-TOCSY. All the spectra were calibrated with acetone as internal (1H 2.225 ppm; 13C 31.45 ppm), acquired with Topspin 2.0 software (Bruker), processed and studied with Topspin 3.6. Details on execution of the spectra are given in the Supporting Information.

### Example 1.9. Attenuated Total Reflectance/FT- Infrared Spectroscopy (ATR FTIR). Thermogravimetric analysis

The structural-functional groups of the EPSₚ were detected using Attenuated Total Reflectance/FT-Infrared Spectroscopy (ATR-FTIR). IR spectra were obtained using a Perkin Elmer BX-FTIR spectrometer coupled with an ATR accessory, MIRacleTM-ATR from PIKE Technologies and interferograms were obtained from 32 scans at a 4 cm⁻¹ with a resolution from 400 to 4000 cm⁻¹.

Thermogravimetric analysis (TGA) of the polymer was done using a TGA Q-500 (Perkin-Elmer). The heating rate for the dynamic conditions was 10°C/min, and the nitrogen flow was maintained constant at 60 mL/min.

### Example 1.10. Scanning Electronic Microscopy (SEM)

Scanning electronic microscopy micrographs were obtained using a Philips XL 30 scanning electron microscope operating in conventional high-vacuum mode at an accelerating voltage of 25 kV. Previously, EPS was coated with a 3 nm thick gold/palladium layer.

### Example 1.11. Cell lines and viruses

Vero, HOG, MeWo, Huh7 and Hela cell lines were propagated in DMEM supplemented with 10% FBS, penicillin (50 U/mL) and streptomycin (50 µg/mL) at 37°C in an atmosphere of 5% CO₂, as described. The Jurkat cell line was cultured in RPMI 1640 medium supplemented with 10% FBS, 2 mM glutamine, 1 mM sodium pyruvate, 10 mMHepes, and 100 mg/mL each of penicillin and streptomycin as described.

In this study, Herpes simplex virus HSV-1 K26GFP (a recombinant virus obtained by fusing GFP to the HSV-1 capsid protein VP26) and HSV-2, Pseudorabies Virus (PRV), XGF-N (virus that expresses the EGFP protein under the control of the gG promoter), Vesicular stomatitis virus (VSV-GFP) and Minute virus of mice (MVM) and HCoV-229E-GFP viruses were tested. Herpesviruses were propagated and titrated on Vero cells. The virus HSV-1 (KOS) gL86, a β-galactosidase-expressing version of KOS strain, was used to monitor the viral entry.

### Example 1.12. Viral infections

To evaluate the effect of EPSp on viral infections, we plated cells in 24-well plates, with or without glass coverslips and, 24 hours later, confluent monolayers were infected with a mixture of viruses and EPSp. To prepare the amount necessary for 10 wells, the virus was incubated or mock-incubated in a microcentrifuge tube with 10 µl of EPSp 1 mg/ml (1 µl of EPSp per well) in serum-free DMEM. The viral inoculum corresponded to an m.o.i. of 0.5 TCID₅₀/ml. Then, we adjusted the final volume up to 30 µl and left the tube for 1 h at 37°C in a CO₂ incubator. After that, we added 2 ml of serum-free DMEM to the tube containing the viral inoculum+EPSp, washed the cells with serum-free DMEM, and infected them with 200 µl per well of the mixture viral inoculum+EPSp (therefore, the final concentration of EPSp in the wells was 5 µg/ml). After 1 hour of viral adsorption, the inoculum was withdrawn, and the cells were washed twice with serum-free DMEN. Finally, cells were incubated in DMEM 10% FBS for 24 hours. A control without EPSp was included. The effect of EPSp on viral infection was evaluated either by immunofluorescence, flow cytometry or quantification of viral production. Viral titter was quantified by an endpoint dilution assay determining the 50% tissue culture infective dose (TCID₅₀) in Vero cells.

### Example 1.13. Viral entry

To determine viral entry, we used the recombinant HSV-1 (KOS) gL86, which expresses beta-galactosidase upon entry into cells. Confluent monolayers of Vero cells plated in 96-well tissue culture dishes were infected at a m.o.i. of 10 with HSV-1 gL86 treated or mock-treated with two-fold serial dilutions of EPSp: 10, 5, 2.5 and 1.25 µl per well as described above. After 1 hour of viral adsorption, the inoculum was withdrawn, and the cells were washed twice with serum-free DMEN. After 6 h p.i., the beta-galactosidase activity was analysed at 410 nm in a microplate reader.

### Example 1.14. Immunofluorescence microscopy

Cells grown on glass coverslips were fixed in 4% paraformaldehyde and processed for immunofluorescence. Images were obtained using an LSM510 META system (Carl Zeiss) coupled to an inverted Axiovert 200 microscope.

### Example 1.15. Flow Cytometry Analysis

To perform FACS analysis, cells were dissociated by incubation for 1 minute in 0.05% trypsin/0.1% EDTA (Invitrogen) at room temperature and washed and fixed in 4% paraformaldehyde for 15 minutes. Then, cells were rinsed and resuspended in PBS. Cells were analysed using a FACSCalibur Flow Cytometer (BD Biosciences).

### Example 1.16. In vivo acute toxicity of EPSp

To evaluate the toxicity of the EPSp produced by *B. licheniformis* IDN-EC *in vivo,* the Balb/c mouse model was used. Twenty male Balb/c mice (21-27 days) were purchased from Charles River Laboratories España and maintained at the Animal Facility of the Centro de Biología Molecular Severo Ochoa (CBMSO, CSIC-UAM, Madrid, Spain). After 2 weeks of acclimation, mice were randomly distributed in 4 cages of 5 individuals and inoculated or mock-inoculated intraperitoneally with 100 µl of different concentrations of EPSp (6, 60 and 600 µg per animal) diluted in isotonic saline solution (NaCl 0.9% w/v) from FisioVet (B. Braun VetCare, Barcelona, Spain). The control consisted of 100 µl of the same saline solution. After injection of the acute dose of EPSp, mice were allowed free access to food and water and monitored daily for mobility, mortality and behavioural changes. At day 14, mice were sacrificed by CO₂ and exsanguinated by cardiac puncture to obtain whole blood, in order to analyse their blood profiles and counts. Several parameters were analysed to monitor renal, hepatic and immunologic basic profiles. For the biochemical analyses, urea, total protein, alanine aminotransferase (ALT) and bilirubin levels were studied. For hematologic analysis, the percentage of lymphocytes and segmented neutrophils was measured, as well as the WBCs count. The body weight gain from the day 0 (inoculation) to the day 14 (sacrifice) was also quantified, to exclude a weight loss or failure to gain weight that would be indicative of toxicity.

### Example 1.17. Statistical analysis

Student's t-test was used to determine differences between groups. All data are represented as mean ± standard deviation.

### Example 1.18. Ethics statement

This study was carried out in strict accordance with the European Commission legislation for the protection of animals used for scientific purposes (directives 86/609/EEC and 2010/63/EU). Mice were maintained under specific pathogen-free conditions at the CBMSO (CSIC-UAM) animal facility. The protocol for the treatment of the animals was accepted by the "Comité de Ética de la Investigación" of the Universidad Autónoma of Madrid, Spain and approved by the "Consejeria General del Medio Ambiente y Ordenación del Territorio de la Comunidad de Madrid" (PROEX 148/15). Animals had unlimited access to food and water, and at the conclusion of the studies they were euthanized in a CO₂ chamber, with every effort made to minimize their suffering, followed by exsanguination by cardiac puncture to obtain whole blood.

### Example 2. Results

### Example 2.1. Biodegradation, cell growth, pH, and EPS production

The growth of *B. licheniformis* IDN-EC, medium biodegradation, pH values and exopolymer production (EPS) at 45°C, are shown in **Figure 1A****.** Cellular growth peaked (9,35 log cfu / ml) after 30 h. In this moment, the strain completely biodegraded the glucose as a carbon source. The maximum production of EPS, 60 mg/ L, occurred after 42 h. During the process, no acute pH descent was detected, as the acidification of the medium was very low (from pH 7 to just 6.6).

### Example 2.2. Characterization of exopolymer

The results of the obtained fraction from the purified exopolymer was named as EPSp. The mass spectrum of the polymer indicated that it had an approximate molecular weight of 5kD (**Figure 1B**). HLPC analysis of EPSp showed that this was formed by galactose and glucosamine with a molar ratio of 3:1.

The mass spectrum of the polymer indicated that it had an approximate molecular weight of 5kD (**Figure 1B**). HLPC and HPLC/MSMS analysis of EPSp, showed that this was a formed by galactose and glucosamine with a molar ratio of 3:1.

Using NMR, the presence of monosaccharides of α conformation was confirmed. In particular the main sugar αGalactose was substituted in position 6. The minority sugar was identified as αGlcNH₂, no products of acetylation were observed.

In addition to this, signals of glycerol esters were identified with different substitution grades (in positions 1-2, 1-3 and/or 1-2-3). Experiments of heteronuclear correlation 1H-31P-HMBC were also conducted. These confirmed the presence of phosphate groups linked to the glycerol forming a polyglycerol phosphate chain. It was determined that the phosphate groups linked the CH₂-OH groups in positions 1 and 3, leaving the two CH-OH groups in the central positions free or glycosylated with the aforementioned sugars. The residues of αGalactose were linked to glycerol with one of the free CH2-OH groups and also bonded with another phosphate group, so these would be in terminal positions. The residues of αGlcNH2 are linked to the glycerol phosphate in positions 1 and 3. Free groups of CH-OH linked to two phosphate bonded positions were also detected.

The position 6 of αGalactose is substituted. It is proposed that this is by a phosphate group although due to the overlap with other glycerol signals it cannot be confirmed. This phosphate could be a point of elongation of the polyglycerol chain.

With this information it is proposed that the composition of the majority products of the samples is the following:

Chain of polyglutamic acid, of no determined length, this being the compound with the largest molecular weight

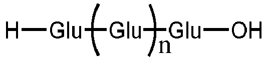

-Chain of polyglycerol phosphate with substitutions of αGalactose in terminal positions and αGlcNH2

Due to the proportion Gal/GlcNH2 (approximately 3:1) and taking into account the terminal position of the αGal, it is possible that some chains do not present αGlcNH2.

The FT-IR spectroscopic analysis was applied to research the polar bonds and vibrations of molecules between the different atoms. The results showed (**Figure 3A**) the presence of the characteristic bands of poly glutamic acid(γ-PGA). The IR spectra of the EPSp of *B. licheniformis* IDN-EC exhibited a broad peak at around 3270 cm ⁻¹ (range 3600-3200 cm ⁻¹) for O-H stretching vibration and the peak at 2924 cm⁻¹ was associated to an amine group. The peak at 1582 cm⁻¹ was associated to the deformation vibration of an amide group. The band at 1399 cm ⁻¹ was assigned to the group C=O whereas the peak at 1052 cm⁻¹ was associated to the C-N group. On the other hand,polyfosfate groups were assigned to the presence of characteristic bands at 1219cm ⁻¹, (range 1200-900)cm⁻¹).

Thermogravimetric analysis (TGA) was used to investigate the thermal stability in the inert atmosphere of EPSp obtained from *B. licheniformis* IDN-EC (**Figure 3B**). The EPSp degradation process took place by reduction of average molecular mass weight. Decomposition of exopolymer started at 242.4°C and 36% of weight loss was observed at 357.41°C. The differential scanning calorimetry (DSC) for EPSp showed exothermic peak for crystallization temperature (Tc) at 272.66.77 °C and the other two peaks formelting temperatures at Tm1 = 356,79 °C and Tm2 = 423,90 °C. The EPSp was highly crystalline. Therefore, it was a biopolymer very thermostable.

The morphology EPSp obtained from *B. licheniformis* IDN-EC was studied using scanning electron microscopy (SEM) (**Figure 3C**). A three-dimensional structure was observed with structural units in the form of thin scales of different sizes intertwined with fine fibers, resulting in a structure of natural scaffolds.

### Example 2.3. Effect of EPSp on herpesvirus infection

The antiviral activity of the exopolymer was assessed using the procedures described in Materials and Methods (Viral infections section). The effects of the EPSp on the HSV-1 K2GFP infection of Vero cells are shown in **Figure 4****.** The immunofluorescence assays showed an almost complete disappearance of the GFP signal in cells infected with EPSp-treated virus at 24 h p.i. with a 5µg/ml dose (**Figure 4A**). To quantify the effect of exopolymer on viral yield, progeny virus was titrated to determine the TCID₅₀/ml. After 24 h p.i., viral yield in Vero cells infected with EPSp-treated virus decreased around 5 orders of magnitude -compared to cells infected with mock-treated virus-, to become practically undetectable (**Figure 4B**).

To investigate whether the decrease in viral yield was due to a decrease in viral entry, Vero cells were infected with the recombinant HSV-1 (KOS) gL86, which expresses beta-galactosidase upon entry into cells. Confluent monolayers of Vero cells plated in 96-well tissue culture dishes were infected at a m.o.i. of 10 with HSV-1 gL86 treated or mock-treated with two-fold serial dilutions of EPSp as described in Materials a Methods (Viral entry section). After 6 hours p.i., the beta-galactosidase activity finding a significant (p<0.05) dose dependent decrease of absorbance in cells treated with EPSp (**Figure 4C**), compared to the control mock-treated cells.

Experiments performed with the rest of cell lines, HOG (**Figure 5A**), Mewo (**Figure 5B**), Hela (**Figure 5C**) and Jurkat (**Figure 5D**), showed similar results: immunofluorescence assays showed a drastic decrease of GFP signal in cells infected with EPSp-treated virus at 24 hours p.i. and, in addition, viral progeny decreased around 4-5 orders of magnitude (depending on the infectivity of the cell lines) in cells infected with EPSp-treated virus compared to cells infected with mock-treated HSV-1 K26GFP (**Figure 5E**).

To analyse whether the results previously shown could be extrapolated to other herpesviruses, Vero cells were infected with HSV-2 and PRV-GFP XGF-N (**Figure 6**) at a m.o.i. of 0.5. Infection with HSV-2 (**Figure 6A**) treated with EPSp at 5 µg/ml showed only a moderate decrease compared to mock-treated control. However, with HSV-2 treated with EPSp at 10 µg/ml, infection drastically decreased. On the other hand, infections with PRV-GFP XGF-N at an m.o.i. of 0.5 yielded similar results at 24 hours p.i., although the effect of EPSp on PRV infection was less pronounced (**Figure 6B**). Thus, infection with PRV-GFP XGF-N treated with EPSp at 10 µg/ml did not caused any observable effect compared to mock-treated control and, to inhibit infection, a dose of EPSp at 20 µg/ml was needed (**Figure 6B**). Similarly, quantification of viral production with HSV-2and PRV-GFP XGF-N, both treated with same doses EPSp (20 µg/ml) showed a decrease about 4 and 3 orders of magnitude respectively (**Figure 6C-D**).

### Example 2.4. Effect of EPSp on other enveloped (VSV) and unenveloped (MVM) viruses

To analyse whether the results above described could be extrapolated to another enveloped virus, cells were infected with VSV-GFP at a m.o.i. of 0.5. As shown in **Figure 7A****,** the decrease of VSV infection was notice able with an EPSp dose of 20 µg/ml. In addition, flow cytometry analysis showed a 4-fold reduction in viral-GFP signal of cells infected with VSV-GFP treated with EPSp 20 µg/ml when compared to non-treated control, and 2-fold reduction when compared to the 5 µg/ml EPSp treatment (**Figure 7B**).

Finally, the EPSp was tested on a non-enveloped virus. Thus, Hela cells were infected with EPSp-treated or mock-treated MVM and, 24 hours p.i., cells were processed for immunofluorescence analysis with an anti-VPs MVM (Minute virus of mice) polyclonal antibody. No change in viral-associated signal was observed in immunofluorescence images, even at an EPSp concentration of 25 µg/ml, suggesting that MVM infection was not affected by the polymer (**Figure 8**).

### Example 2.5. Effect of EPSp on coronavirus infection

The antiviral activity of the exopolymer was assessed also in coronavirus, single-stranded enveloped viruses with positive polarity. To test the effect of EPSp on coronavirus infection, Huh7 cells were infected at an m.o.i. of 0.1 with HCov229-E-GPF pre-treated or mock pre-treated with EPSp (5 and 20 µ/ml) and cultured for 48 hours at 37°C in a CO₂ incubator. Immunofluorescence images showed a drastic decrease of infection in Huh7 cells infected with HCOV229-E-GFP virus treated with EPSp compared to control (**Figure 9**).

### Example 2.6. Acute toxicity study of EPSp in mice

To establish the safety of the EPSp *in vivo,* twenty male Balb/c mice were inoculated or mock-inoculated with different single doses of the EPSp as described in Materials and Methods (*In vivo* acute toxicity of EPSp section). No behavioural changes, morbidity or deaths were observed in any animal of the control and the experimental groups after being injected with the EPSp. No toxic signs were observed such as hypothermia, weakness, diarrhea or ataxia, nor signs of acute pain or distress, during the 14 days observation period. In addition, the weight of each animal in the three experimental groups showed no significant changes compared to the controls. From the levels of urea (**Figure 10A**), total protein (**Figure 10B**), alanine aminotransferase (ALT) (**Figure 10C****)** and bilirubin (**Figure 10**) in blood samples taken from EPSp-treated mice at 14 days after the intraperitoneal injection, there were no significant changes in any of the parameters between the control and experimental groups. The percentage of lymphocytes (**Figure 10E**), the white blood cells (WBCs) count (**Figure 10F**) and segmented neutrophils (**Figure 10G**) corresponding to the experimental groups, also showed no significant changes compared to the controls. In addition, the weight of each animal in the three experimental groups showed no significant changes compared to the controls (**Figure 10H**).

## Claims

1. *In vitro* method for obtaining a polymer with antiviral activity which comprises:
a. Incubating the bacterial strain *Bacillus licheniformis* **characterized by** the 16S rDNA of SEQ ID NO: 1 in suitable growth media;
b. Centrifugating the cultures obtained in step a);
c. Precipitating the supernatant obtained in step b);
d. Collecting the precipitate obtained from step c);
e. Dialyzing; and
f. Freeze drying.

2. *In vitro* method, according to claim 1, wherein the incubating step a) comprises:
a. Transferring the strain into flasks filled with minimal growth medium comprising: 6.8 g/L Na₂HPO4, 3 g/L KH₂PO4, 1 gr/L NH₄Cl, 0.625 gr/L NaCl, 0.49 gr/L MgSO₄ - 7H₂O, 0.011 gr/L CaCl₂ and 4 gr/L Glucose,
b. Adjusting the pH to 7.0.
c. Incubating in a rotary at 45°C and 110 rpm for 24 hours.
d. Inoculating 10 ml of the minimal growth medium with the strain into flasks filled with 100 ml of minimal growth medium with Glucose supplementation.
e. Incubating the flasks in a rotary at 45°C and 110 rpm for 40 hours.

3. *In vitro* method, according to any of the previous claims, wherein the centrifugating step b) is carried out at 12000-14000 x g for 30 minutes at 4°C.

4. *In vitro* method, according to any of the previous claims, wherein the precipitating step c) is carried out with cold ethanol.

5. *In vitro* method, according to any of the previous claims, wherein the collecting step d) is carried out by centrifugation at 12000-14000 x g for 30 minutes at 4°C.

6. *In vitro* method, according to any of the previous claims, wherein the dialyzing step d) is carried out at 4°C with Milli-Q water for 48h.

7. *In vitro* method, according to any of the previous claims, wherein the freeze-drying e) is carried out by lyophilization for 48h.

8. Use of the bacterial strain *Bacillus licheniformis* **characterized by** the 16S rDNA of SEQ ID NO: 1 for obtaining a polymer with antiviral activity.

9. Polymer obtainable by the method of any of the claims 1 to 7.

10. Polymer, according to claim 9, comprising substantially a chain of polyglycerol phosphate with substitutions of Galactose and Glucosamine in a proportion 3:1 and a basic repeating unit of polyglutamic acid, wherein the molecular weight of the polymer is between 4 to 6 kD.

11. Polymer, according to any of the claims 9 or 10, **characterized in that** it is produced by culturing the bacterial strain *Bacillus licheniformis* **characterized by** the 16S rDNA of SEQ ID NO: 1 under conditions conducive for the production of the polymer.

12. Pharmaceutical composition comprising the polymer of any of the claims 9 to 11 and optionally pharmaceutically acceptable excipients and/or carriers.

13. Pharmaceutical composition, according to claim 12, for use a medicament.

14. Pharmaceutical composition for use, according to claim 13, in the treatment of a viral infection caused by an enveloped virus.

15. Pharmaceutical composition for use, according to claim 12, in the treatment of a viral infection caused by an enveloped virus selected from the group comprising: Herpes simplex virus (HSV-1 or HSV-2), Pseudorabies virus (PRV), Vesicular stomatitis virus (VSV), or Coronavirus.


SEQUENCE LISTING

<110>  Universidad Autónoma de Madrid

<120>  ANTIVIRAL COMPOSITION

<130>  905 089

<160>  1

<170>  PatentIn version 3.5

<210>  1
<211>  1510
<212>  DNA
<213>  Artificial sequence

<220>
<223>  Artificial sequence

<400>  1
agagtttgtc ctggctcagg acgaacgctg gcggcgtgcc taatacatgc aagtcgagcg      60

gaccgacggg agcttgctcc cttaggtcag cggcggacgg gtgagtaaca cgtgggtaac     120

ctgcctgtaa gactgggata acttcgggaa accggggcta ataccggatg cttgattgaa     180

ccgcatggtt caattataaa aggtggcttt cagctaccac ttgcagatgg acccgcggcg     240

cattagctag ttggtgaggt aacggctcac caaggcgacg atgcgtagcc gacctgagag     300

ggtgatcggc cacactggga ctgagacacg gcccagactc ctacgggagg cagcagtagg     360

gaatcttccg caatggacga aagtctgacg gagcaacgcc gcgtgagtga tgaaggtttt     420

cggatcgtaa aactctgttg ttagggaaga acaagtgccg ttcgaatagg gcggcacctt     480

gacggtacct aaccagaaag ccacggctaa ctacgtgcca gcagccgcgg taatacgtag     540

gtggcaagcg ttgtccggaa ttattgggcg taaagcgcgc gcaggcggtt tcttaagtct     600

gatgtgaaag cccccggctc aaccggggag ggtcattgga aactggggaa cttgagtgca     660

gaagaggaga gtggaattcc acgtgtagcg gtgaaatgcg tagagatgtg gaggaacacc     720

agtggcgaag gcgactctct ggtctgtaac tgacgctgag gcgcgaaagc gtggggagcg     780

aacaggatta gataccctgg tagtccacgc cgtaaacgat gagtgctaag tgttagaggg     840

tttccgccct ttagtgctgc agcaaacgca ttaagcactc cgcctgggga gtacggtcgc     900

aagactgaaa ctcaaaggaa ttgacggggg cccgcacaag cggtggagca tgtggtttaa     960

ttcgaagcaa cgcgaagaac cttaccaggt cttgacatcc tctgacaacc ctagagatag    1020

ggcttcccct tcgggggcag agtgacaggt ggtgcatggt tgtcgtcagc tcgtgtcgtg    1080

agatgttggg ttaagtcccg caacgagcgc aacccttgat cttagttgcc agcattcagt    1140

tgggcactct aaggtgactg ccggtgacaa accggaggaa ggtggggatg acgtcaaatc    1200

atcatgcccc ttatgacctg ggctacacac gtgctacaat gggcagaaca aagggcagcg    1260

aagccgcgag gctaagccaa tcccacaaat ctgttctcag ttcggatcgc agtctgcaac 1320

tcgactgcgt gaagctggaa tcgctagtaa tcgcggatca gcatgccgcg gtgaatacgt 1380

tcccgggcct tgtacacacc gcccgtcaca ccacgagagt ttgtaacacc cgaagtcggt 1440

gaggtaacct tttggagcca gccgccgaag gtgggacaga tgattggggt gaagtcgtaa 1500

caaggtaacc 1510